# EUROPEAN PATENT SPECIFICATION

(11) **EP 2 823 806 B1**
(45) Date of publication and mention of the grant of the patent: **10.05.2017**
(21) Application number: 13758653.3
(22) Date of filing: 06.03.2013
(51) Int. Cl.: A61K 8/36, A61K 8/39, A61K 8/73, A61Q 19/10, C11D 1/06, C11D 1/52, C11D 1/94, C11D 3/22, C11D 17/08

(54) **SKIN WASHING COMPOSITION**
HAUTWASCHZUSAMMENSETZUNG
COMPOSITION DE LAVAGE DE LA PEAU

(30) Priority: 07.03.2012 JP 2012050195
(43) Date of publication of application: 14.01.2015
(73) Proprietor: Kao Corporation, Chuo-Ku Tokyo 103-8210 (JP)
(72) Inventor: TAKEUCHI, Hiroki, Tokyo 131-8501 (JP); YAMAMOTO, Tomoyuki, Tokyo 131-8501 (JP); ANUMANSIRIKUL, Nattaporn, Bangkok 10330 (TH)
(74) Representative: Hoffmann Eitle
(86) International application number: PCT/JP2013/056159
(87) International publication number: WO 2013/133328

(56) References cited:
- EP-A2- 1 982 692
- DE-A1-102004 027 323
- JP-A- H0 625 695
- JP-A- S6 197 395
- JP-A- H05 112 795
- JP-A- 2005 112 946
- JP-A- 2011 140 485
- JP-A- 2011 178 681
- US-A- 5 180 584
- SHUCHI - KAN'YO GIJUTSUSHU (KESHOHIN OYOBI RUIJIHIN) 21 August 1984, page 20, XP008174531
- KESHOHIN HANDBOOK 01 November 1996, pages 163 - 165, XP008174613

## Description

### [Field of the Invention]

The present invention relates to a skin cleansing composition.

### [Background of the Invention]

In order to wash the skin gently, there have been used cleansing compositions comprising cleanser bases mild to the skin.

For example, it is known that excellent foam qualities and foam having detergency can be formed by using a combination of a low concentration of aqueous solution of fatty acid soap, and a polyoxyethylene alkyl ether acetic acid and/or a salt thereof which is perceived to be mild to the skin (Patent Publication 1). Also, a cleansing composition comprising a higher fatty acid salt, an amidopropyl betaine surfactant, and a hydroxypropyl methyl cellulose produces a good lather and has high detergency, but is not irritating to the skin during and after washing. Moreover, after using this cleansing composition, a fresh feeling upon use can be obtained (Patent Publication 2).

DE 10 2004 027323 concerns a cosmetic preparation such as a shaving soap, comprising alkylethercarboxylate (a), ethoxylated glycerine (b), one or more soaps of 12-18C fatty acids (c) and water. A pressure gas container comprising the preparation is described as well.

EP 1 982 692 describes a skin cleansing composition, which contains a polyoxyethylene alkylether sulfate, a polyoxyethylene alkylether carboxylate, and a cationic group-containing polymer having a cationic charge density of not less than 4.5 meq/g, and/or polyvinylpyrrolidone. The skin cleansing composition is said to be excellent in foamability and foam quality, and to provide a good stop feeling during rinsing and a refreshed touch feeling after towel blotting.

US 5,180,584 relates to washing compositions based on insoluble silicones, containing in an aqueous medium: (a) a silicone insoluble in the said medium; and (b) at least 7% by weight of an alkyl ether carboxylate surfactant of a specific formula (I), with the ratio of the compound of formula (I)/silicone being higher than 1.

### Citation List

### [Patent Publication]

[Patent Publication 1] JP-A-2008-115094
[Patent Publication 2] JP-A-2002-167324

### [Summary of the Invention]

The present invention relates to a skin cleansing composition, comprising the following components (A), (B), (C) and (D):
(A) from 0.5 to 6% by mass of an alkyl ether carboxylic acid or a salt thereof represented by the formula (1):

   R¹-O-(CH₂CH₂O)-CH₂-COOM (1)

   wherein, R¹ represents an alkyl group having 4 to 22 carbon atoms, n represents a number of from 0 to 20, the average value of n being 2.8 to 3.4, and M represents a hydrogen atom, alkali metal, alkaline earth metal, ammonium, or organic ammonium,
   wherein, R¹ has an average carbon number of from 10.8 to 12.8,
   and wherein, the alkyl ether carboxylic acid or the salt thereof contains a component in which n = 0 in an amount of more than 9.6% by mass and 27% by mass or less, and a component in which n = 1 and a component in which n = 2 in a total amount of 21% by mass or more and less than 40% by mass,
(B) from 0.1 to 1% by mass of a cellulose having a hydroxyethyl group or a hydroxypropyl group added,
(C) from 3 to 30% by mass of an anionic surfactant other than the component (A), and
(D) water.

### [Detailed Description of the Invention]

In fact, it was difficult to feel the mildness to the skin during washing with the cleansing compositions of Patent Publications 1 and 2.

The present invention relates to a skin cleansing composition which makes the skin feel soft during washing so that the user can be assured of mildness to the skin.

The present inventors have found that a skin cleansing composition which gives the impression of thickness of foam and makes the skin feel soft during washing so that the user can be assured of mildness can be obtained by using an alkyl ether carboxylic acid or a salt thereof having a specific distribution in combination with a specific cellulose derivative and a specific anionic surfactant.

The skin cleansing composition of the present invention produces a good lather and gives the impression of thickness of foam, and makes the skin feel soft during washing so that the user can be assured of mildness.

The alkyl ether carboxylic acid or the salt thereof of the component (A) used in the present invention is represented by the formula (1).

In the formula, R¹ is an alkyl group having 4 to 22 carbon atoms, preferably an alkyl group having 6 to 20 carbon atoms, more preferably an alkyl group having 8 to 18 carbon atoms, even more preferably an alkyl group having 8 to 16 carbon atoms, and further preferably an alkyl group having 10 to 16 carbon atoms. Also, although the alkyl chain of R¹ may be either linear or branched, from the viewpoint of foaming properties, a linear alkyl group is preferred. Also, R¹ has an average carbon number of from 10.8 to 12.8, preferably from 10.8 to 12.5, and more preferably from 12.1 to 12.4. It is preferable that the average carbon number be within the above range since excellent foaming properties, foam qualities, and stability at low temperature are obtained.

Also, R¹ preferably contains two or more alkyl groups, and the content of a component having the alkyl chain length contained therein with the highest content is preferably 55% by mass or more and less than 97% by mass, more preferably from 60 to 95% by mass, and even more preferably from 70 to 95% by mass since excellent volume of foam and foam qualities are obtained.

Also, in the formula, n represents a number of from 0 to 20, and preferably from 0 to 12. It is to be noted that n represents the number of moles of ethylene oxide added, and the average number of moles of ethylene oxide added in the composition of the component (A) (an average value of n) is from 2.8 to 3.4, and preferably from 2.8 to 3.1 since a good lather is produced.

The alkyl ether carboxylic acid or the salt thereof of the component (A) contains, in the formula (1), a component in which n = 0 in an amount of more than 9.6% by mass and 27% by mass or less, preferably from 9.8 to 27% by mass, more preferably from 9.9 to 27% by mass, even more preferably from 9.9 to 16% by mass, and further preferably from 9.9 to 15% by mass. When the content of the component in which n = 0 is within the above range, excellent volume of foam and foam qualities are obtained.

Further, from the viewpoint of foam qualities and volume of foam, the total content of a component in which n = 1 and a component in which n = 2 is 21% by mass or more and less than 40%, preferably from 27 to 37% by mass, more preferably from 27 to 36.5% by mass, and even more preferably from 35 to 36.1% by mass.

Also, in the formula, examples of M include a hydrogen atom; alkali metal such as sodium and potassium; alkaline earth metal such as calcium and magnesium; ammonium; alkanolamine-derived ammonium such as monoethanolamine, diethanolamine, and triethanolamine. Among them, alkali metal is preferred in terms of foaming properties, stability at low temperature, and absence of coloration over time.

In the formula (1), the alkyl ether carboxylic acid or the salt thereof of the component (A) preferably has a mass ratio of the components in which n = 0, 1, 2, 3, and 4, (the mass of a component in which n = 0) : (the mass of a component in which n = 1) : (the mass of a component in which n = 2) : (the mass of a component in which n = 3) : (the mass of a component in which n = 4), of 1 : 0.99 to 3.50 : 0.89 to 3.00 : 0.76 to 3.00 : 0.63 to 1.52 in a component having the alkyl chain length contained with the highest content in a composition of R¹ from the viewpoint that foaming properties, detergency, and a feeling of friction during rinsing can be achieved simultaneously.

Also, in the formula (1), it is preferable that the content of a component in which n = 0 be 9.9% by mass or more and less than 12% by mass, and a ratio of (the mass of a component in which n = 0) : (the mass of a component in which n = 1) : (the mass of a component in which n = 2) : (the mass of a component in which n = 3) : (the mass of a component in which n = 4) = 1 : 1.53 to 1.87 : 1.59 to 2.25 : 1.33 to 2.16 : 1.14 to 1.52 in a component having the alkyl chain length contained with the highest content in a composition of R¹, or the content of a component in which n = 0 be from 12 to 17% by mass and a ratio of (the mass of a component in which n = 0) : (the mass of a component in which n = 1) : (the mass of a component in which n = 2) : (the mass of a component in which n = 3) : (the mass of a component in which n = 4) = 1 : 0.99 to 1.34 : 0.89 to 1.40 : 0.76 to 1.23 : 0.63 to 1 in a component having the alkyl chain length contained with the highest content in a composition of R¹ from the viewpoint of foam qualities and volume of foam.

Further, in the formula (1), it is preferable that the content of a component in which n = 0 be from 9.9 to 11.5% by mass and a ratio of (the mass of a component in which n = 0) : (the mass of a component in which n = 1) : (the mass of a component in which n = 2) : (the mass of a component in which n = 3) : (the mass of a component in which n = 4) = 1 : 1.58 to 1.84 : 1.72 to 2.17 : 1.49 to 2.00 : 1.14 to 1.52 in a component having the alkyl chain length contained with the highest content in a composition of R¹, or it is preferable that the content of a component in which n = 0 be from 13 to 15% by mass and a ratio of (the mass of a component in which n = 0) : (the mass of a component in which n = 1) : (the mass of a component in which n = 2) : (the mass of a component in which n = 3) : (the mass of a component in which n = 4) = 1 : 1.00 to 1.31 : 0.93 to 1.34 : 0.79 to 1.18 : 0.63 to 1 in a component having the alkyl chain length contained with the highest content in a composition of R¹ from the viewpoint of foam qualities and volume of foam.

It is preferable that, in the component (A), in the formula (1), R¹ be an alkyl group having 4 to 22 carbon atoms, R¹ has an average carbon number of from 10.8 to 12.8, and the content of a component having an alkyl chain length contained therein with the highest content be 55% by mass or more and less than 97% by mass, and further, n represents a number of from 0 to 20 and the average value of added moles n be from 1.5 to 10, more preferably from 2.5 to 6.4, and a component in which n = 0 be contained in an amount of from 9.8 to 27% by mass, and a component in which n = 1 and a component in which n = 2 be contained in a total amount of from 27 to 37% by mass. Also, as M in the formula, a hydrogen atom, sodium, potassium, and ammonium are preferred. A cleansing composition comprising the alkyl ether carboxylic acid or the salt thereof having the aforementioned configuration can achieve accelerated foaming.

It is preferable that, in the component (A), in the formula (1), R¹ be an alkyl group having 8 to 16 carbon atoms, R¹ has an average carbon number of 10.8 to 12.5, and the content of a component having the alkyl chain length contained therein with the highest content be from 60 to 95% by mass, and further, n represents a number of from 0 to 20 and the average value of n be from 2.8 to 3.4, and a component in which n = 0 be contained in an amount of from 9.9 to 16% by mass and a component in which n = 1 and a component in which n = 2 be contained in a total amount of from 27 to 36.5% by mass. Also, as M in the formula, a hydrogen atom, sodium, potassium, and ammonium are preferred. A cleansing composition comprising the alkyl ether carboxylic acid or the salt thereof having the aforementioned configuration can have improved volume of foam and foam qualities.

It is preferable that, in the component (A), in the formula (1), R¹ be an alkyl group having 10 to 16 carbon atoms, R¹ has an average carbon number of from 12.1 to 12.4, and the content of a component having the alkyl chain length contained therein with the highest content be from 70 to 95% by mass, and further, n represents a number of from 0 to 20 and the average value of n be from 2.8 to 3.1, and a component in which n = 0 be contained in an amount of from 9.9 to 15% by mass, and a component in which n = 1 and a component in which n = 2 be contained in a total amount of from 35 to 36.1% by mass. Also, as M in the formula, a hydrogen atom, sodium, potassium, and ammonium are preferred. A cleansing composition comprising the alkyl ether carboxylic acid or the salt thereof having the aforementioned configuration can have improved volume of foam and foam qualities.

It is to be noted that in the component (A) of the present invention, the distribution of the alkyl chain length of R¹, the average alkyl chain length of R¹, the amount of a component in which n = 0, the average number of added moles n, and a mass ratio of the components in which n = 0, 1, 2, 3, and 4 are obtained as follows from the gas chromatographic analysis of the alkyl ether carboxylic acid represented by the formula (1).

### [Distribution of the alkyl chain length of R¹]

From the peak areas obtained by gas chromatography, a peak area of each alkyl chain length corresponding to n = 0 mole was obtained, and setting the sum of the peak areas thus obtained at 100, the percentage of the distribution of each alkyl chain length was calculated. Similar calculation was carried out also as to n = 1 to 3 moles, and the percentage values of the distribution of each alkyl chain length corresponding to n = 0 to 3 moles were averaged out, whereby the distribution of the alkyl chain length of R¹ was obtained (from this, the alkyl group component contained in the largest amount in the composition of R¹ can be specified).

### [Average alkyl chain length of R¹]

From the distribution of the alkyl chain length of R¹ obtained as above, the proportion of each component was obtained, which was multiplied by the number of carbon atoms of the corresponding alkyl chain length, and the resulting values were summed up. The value thus obtained was used as an average alkyl chain length.

### [Amount of a component in which n = 0, total content of a component in which n = 1 and a component in which n = 2]

In the composition of R¹, the alkyl chain length contained therein with the highest content was specified, and the peak areas of the component having alkyl chain length of the highest content corresponding to n = 0 to 10 were added up by gas chromatography. By setting the total amount thus obtained at 100%, the amount of a component in which n = 0, the total content of a component in which n = 1 and a component in which n = 2 were calculated.

### [Average number of added moles n]

In the composition of R¹, the alkyl chain length of the highest content was specified, and the peak areas of the component having the alkyl chain length of the highest content corresponding to n = 0 to 10 were added up by gas chromatography (the amount of a component in which n is 11 or more was so small that it was excluded from the calculation). By setting the total amount thus obtained at 1, each proportion of n = 0 to 10 was obtained. The resulting proportion was multiplied by each number of added moles, and the sum of the resulting values was used as the average number of added moles n.

### [Mass ratio of the components in which n = 0, 1, 2, 3, and 4]

As to the mass ratio of each of the components having different numbers of moles of EO added, the distribution of the alkyl chain length of R¹ was obtained from the peak area obtained by gas chromatography by the method described above, and the component having the alkyl chain length of the highest content in the composition of R¹ was specified, and the ratio of each of the components having different numbers of moles of EO added was specified by the area ratio of n = 0, n = 1, n = 2, n = 3, and n = 4 of the component having the alkyl chain length of the highest content.

The alkyl ether carboxylic acid or the salt thereof of the component (A) has the aforementioned composition, and from the viewpoint of foaming and imparting a moist feeling after towel drying, the content thereof is, as acid, 0.5% by mass or more, preferably 1% by mass or more, more preferably 2% by mass or more, and 6% by mass or less, preferably 5% by mass or less, and more preferably 4% by mass or less of the total composition. Also, the content of the component (A) is, as acid, from 0.5 to 6% by mass, more preferably from 1 to 5% by mass, even more preferably from 1 to 4% by mass, and further preferably from 2 to 4% by mass of the total composition.

The cellulose of the component (B) used in the present invention is a cellulose having a hydroxyethyl group or a hydroxypropyl group added. That is, the cellulose of the component (B) is a cellulose in which one or more hydrogen atoms in the hydroxyl group are replaced by a hydroxyethyl group or a hydroxypropyl group. The cellulose of the component (B) may have a substituent other than the above-mentioned ones.

Specific examples include hydroxyethylcellulose, hydroxypropylcellulose, hydroxyethyl methyl cellulose, and hydroxypropyl methyl cellulose.

These celluloses are obtained by reacting caustic soda with a cellulose to obtain alkali cellulose, and then allowing a substance such as methyl chloride, monochloroacetic acid, ethylene oxide, and propylene oxide to act on the alkali cellulose thus obtained to thereby replace the hydrogen atom in the hydroxyl group of the cellulose.

From the viewpoint of the foam retentivity-improving effect, the average degree of substitution in the cellulose of the component (B) is larger than 0, more preferably 0.5 or more, and preferably 3 or less, and more preferably 2 or less. Also, from the viewpoint of dissolution into the cleansing composition and foam retentivity, the average molecular weight is preferably 200,000 or more, more preferably 350,000 or more, even more preferably 500,000 or more, and further preferably 650,000 or more, and preferably 3,000,000 or less, more preferably 2,000,000 or less, and even more preferably 1,600,000 or less.

It should be noted that, in the present invention, the average degree of substitution is obtained by NMR, and the average molecular weight indicates the weight-average molecular weight.

As the component (B), hydroxyethylcellulose and hydroxypropyl methyl cellulose are preferable, and one having an average degree of substitution of from 0.5 to 2 and an average molecular weight of from 650,000 to 1,600,000 is preferable.

Also, as the component (B), for example, as hydroxyethylcellulose, commercial products such as CELLOSIZE QP52000H (manufactured by The Dow Chemical Company) and HEC DAICEL SE400, SE500, SE600, SE850, and SE900 (all are manufactured by Daicel FineChem Ltd.); and as hydroxypropyl methyl cellulose, commercial products such as METOLOSE 60SH and 65SH (both are manufactured by Shin-Etsu Chemical Co., Ltd.), and BENECEL E50, E4M, E10M, F4MC, K99C, K4M, K15M, K35M, K100M, and K200M (all are manufactured by Ashland Inc.) can be used.

The component (B) can be used alone or in combination of two or more thereof, and from the viewpoint of foam retentivity and imparting the impression of thickness of foam, the content thereof is 0.1% by mass or more, more preferably 0.2% by mass or more, even more preferably 0.3% by mass or more, and 1% by mass or less, preferably 0.8% by mass or less, and more preferably 0.5% by mass or less of the total composition. Also, the content of the component (B) is from 0.1 to 1% by mass, preferably from 0.2 to 0.8% by mass, more preferably from 0.3 to 0.8% by mass, and even more preferably from 0.3 to 0.5% by mass of the total composition.

As the anionic surfactant of the component (C), one which is other than the component (A) and is used in common skin cleansers can be used. Examples thereof include fatty acid salts, alkyl (ether) sulfate, allyl (ether) sulfate, alkylbenzene sulfonate, alkane sulfonate, olefin sulfonate, alkyl ether sulfonate, glyceryl ether sulfonate, α-methyl ester sulfonate, sulfofatty acid salts, alkyl sulfate, fatty alcohol ether sulfate, glyceryl ether sulfate, hydroxy mixed ether sulfate, monoglyceride (ether) sulfate, fatty acid amide (ether) sulfate, monoalkyl sulfosuccinate, dialkyl sulfosuccinate, monoalkyl sulfosuccinamate, dialkyl sulfosuccinamate, sulfotriglyceride, amide ether carboxylic acid salts and salts thereof, fatty acid isethionate, fatty acid sarcosinate, and fatty acid tauride; N-acylamino acid salts such as acyl lactylate, acyl tartrate, acyl glutamate, and acyl aspartate; alkyl oligoglucoside sulfate, protein fatty acid condensate (wheat-based vegetable products), and alkyl (ether) phosphate.

Among them, a fatty acid salt and alkyl (ether) sulfate are preferable.

As the fatty acid salt, one represented by the formula (2) is preferable:

R²-COOY (2)

wherein, R² represents a linear or branched alkyl group or alkenyl group having 9 to 21 carbon atoms and Y represents alkali metal, ammonium, alkanolamine-derived ammonium, or a basic amino acid.

More specifically, examples thereof include salts of, for example, lauric acid, myristic acid, palmitic acid, stearic acid, isostearic acid, oleic acid, arachic acid, and behenic acid. Examples of the salts of these acids include alkali metal such as potassium; ammonium; ammonium derived from alkanolamine such as monoethanolamine, diethanolamine, and triethanolamine; and basic amino acid such as arginine and lysine.

Also, as the alkyl (ether) sulfate, one represented by the formula (3) is preferable:

R³-O-(CH₂CH₂O)ₚ-SO₃-Z (3)

wherein, R³ represents a linear or branched alkyl group or alkenyl group having 8 to 22 carbon atoms, p represents a number of from 0 to 20, the average number of moles added is from 0 to 3, and Z represents a hydrogen atom or a cation selected from alkali metal, alkaline earth metal, ammonium, alkyl ammonium, alkanol ammonium, and glucammonium.

Alkyl (ether) sulfate is a sulfate monoester of (alkoxyl)alcohol, and this is obtainable by sulfating alcohol, followed by neutralization with bases such as sodium hydroxide and triethanolamine.

In the present invention, in the formula (3), R³ is preferably a linear or branched alkyl group or alkenyl group having 12 to 18 carbon atoms, and more preferably a linear or branched alkyl group or alkenyl group having 12 to 14 carbon atoms.

In the formula (3), p is 0 or the average number of moles added is preferably larger than 0 and 3 or less, more preferably larger than 0 and less than 2, and even more preferably from 0.5 to 1.5.

The component (C) can be used alone or in combination of two or more thereof, and from the viewpoint of the volume of foam, imparting the impression of thickness of foam, and softness of the skin during washing, the content of the component (C) is 3% by mass or more, preferably 8% by mass or more, more preferably 15% by mass or more, even more preferably 18% by mass or more, and 30% by mass or less, preferably 23% by mass or less, and more preferably 21% by mass or less of the total composition. Also, the content of the component (C) is from 3 to 30% by mass, preferably from 8 to 23% by mass, even more preferably from 15 to 23% by mass, and further preferably from 18 to 21% by mass of the total composition.

Also, the present invention can make the skin feel soft during washing so that the user can be assured that the body is gently washed by comprising the components (A), (B), and (C). In the present invention, the mass ratio of the component (A) to the component (C), (A)/(C), is preferably 0.03 or more, more preferably 0.04 or more, and preferably 2 or less, and more preferably 0.25 or less since the impression of thickness of foam can be increased, and the user can be more assured of soft skin and that the body is gently washed. Also, the mass ratio of the component (A) to the component (C), (A)/(C), is preferably from 0.03 to 2, and more preferably from 0.04 to 0.25.

In the present invention, (D) water is used as a solvent. The content thereof is preferably 30% by mass or more, more preferably 65% by mass or more, and preferably 95% by mass or less, more preferably 85% by mass or less, and even more preferably 77% by mass or less of the total composition. Also, the content of the component (D) is preferably from 30 to 95% by mass, more preferably from 65 to 85% by mass, and even more preferably from 65 to 77% by mass of the total composition.

The skin cleansing composition of the present invention can further comprise (E) an amphoteric surfactant, whereby the viscosity of the composition can be increased and the thickness and spreadability of foam can be improved.

As the amphoteric surfactant of the component (E), one which is used in common skin cleansers can be used. Examples thereof include alkyldimethylaminoacetic acid betaine having 12 to 18 carbon atoms such as lauryldimethylaminoacetic acid betaine, alkyldimethylamine oxide having 12 to 18 carbon atoms such as lauryldimethylamine oxide, 2-alkyl-N-carboxymethyl-N-hydroxyethyl imidazolinium betaine, fatty acid amidopropyl betaine having 12 to 18 carbon atoms such as lauric acid amidopropyl betaine, and alkyl hydroxy sulfobetaine having 12 to 18 carbon atoms such as lauryl hydroxy sulfobetaine. Among them, fatty acid amidopropyl betaine having 12 to 18 carbon atoms and alkyl hydroxy sulfobetaine having 12 to 18 carbon atoms are preferable, and lauric acid amidopropyl betaine and lauryl hydroxy sulfobetaine are more preferable.

The component (E) can be used alone or in combination of two or more thereof, and from the viewpoint of easy spreading of foam during washing, the content thereof is preferably 0.5% by mass or more, more preferably 1.5% by mass or more, and preferably 5% by mass or less, more preferably 3.5% by mass or less, and even more preferably 2% by mass or less of the total composition. Also, the content of the component (E) is preferably from 0.5 to 5% by mass, more preferably from 1.5 to 3.5% by mass, and even more preferably from 1.5 to 2% by mass of the total composition.

The skin cleansing composition of the present invention can further comprise (F) an alkyl alkanolamide represented by the formula (4): 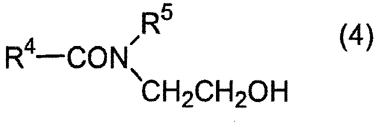 wherein, R⁴CO represents an acyl group having 8 to 20 carbon atoms and R⁵ represents a hydrogen atom, a methyl group, or a hydroxyethyl group,
whereby the viscosity of the composition can be increased and the impression of thickness during the application of the agent can be improved.

In the formula (4), R⁴CO is preferably an acyl group having 8 to 20 carbon atoms, and more preferably an acyl group having 10 to 18 carbon atoms. Also, R⁵ is preferably a hydrogen atom or a methyl group.

The component (F) can be used alone or in combination of two or more thereof, and from the viewpoint of imparting the impression of thickness of liquid, thereby giving a rich feeling during the application of the agent to the skin, the content thereof is preferably 0.2% by mass or more, more preferably 0.5% by mass or more, and preferably 2% by mass or less, and more preferably 1% by mass or less of the total composition. Also, the content of the component (F) is preferably from 0.2 to 2% by mass, and more preferably from 0.5 to 1% by mass of the total composition.

The skin cleansing composition of the present invention may further comprise components used in ordinary cleansers such as surfactants other than the components (A), (C) and (E), humectants, oil components, disinfecting agents, anti-inflammatory agents, antiseptic agents, chelating agents, thickening agents, salts, pearlescent agents, scrub agents, fragrances, cooling agents, dyes, ultraviolet absorbers, antioxidants, and plant extracts.

The skin cleansing composition of the present invention is produced by mixing the blending components by a routine method. The cleansing composition thus obtained may be either a liquid or a solid. When it is a liquid, the viscosity at 25°C as measured by a B-type viscometer (under the conditions of VISCOMETER TVB-10, manufactured by Toki Sangyo Co., Ltd., with No.3 rotor or No.4 rotor, at 30, 12 or 6 rpm, at 30°C after 60 seconds) is preferably from 200 to 80000 mPa·s. The viscosity can be adjusted by appropriately selecting the blending components.

Also, the pH is preferably from 3 to 12, more preferably from 5 to 10.5, and even more preferably from 5 to 10. Also, the degree of pH is measured in each cleansing composition diluted 20-fold with ion exchange water at 25°C.

The skin cleansing composition of the present invention is suitable as, for example, a face wash, a body soap, and a hand soap. The skin cleansing composition of the present invention is more preferable as a body soap.

The skin cleansing method using the skin cleansing composition of the present invention is exemplified as follows. That is, there is provided a method comprising applying an adequate amount of the skin cleansing composition of the present invention to the body, namely the body's skin areas such as face, hands, feet, and torso, lathering up and washing, and then rinsing off using warm water from a shower and the like. It is also possible to apply an adequate amount of the cleansing composition of the present invention to a washing aid such as a towel, a sponge, and a brush, and then lather up and wash.

In connection with the aforementioned embodiments, the present invention further discloses the following compositions, production methods and applications.
<1> A skin cleansing composition, comprising the following components (A), (B), (C) and (D):
   (A) from 0.5 to 6% by mass of an alkyl ether carboxylic acid or a salt thereof represented by the formula (1):

      R¹-O-(CH₂CH₂O)ₙ-CH₂-COOM (1)

      wherein, R¹ represents an alkyl group having 4 to 22 carbon atoms, n represents a number of from 0 to 20, the average value of n being 2.8 to 3.4, and M represents a hydrogen atom, alkali metal, alkaline earth metal, ammonium, or organic ammonium,
      wherein, R¹ has an average carbon number of from 10.8 to 12.8,
      and wherein, the alkyl ether carboxylic acid or the salt thereof contains a component in which n = 0 in an amount of more than 9.6% by mass and 27% by mass or less and a component in which n = 1 and a component in which n = 2 in a total amount of 21% by mass or more and less than 40% by mass,
   (B) from 0.1 to 1% by mass of a cellulose having a hydroxyethyl group or a hydroxypropyl group added,
   (C) from 3 to 30% by mass of an anionic surfactant other than the component (A), and
   (D) water.
<2> The skin cleansing composition according to the aforementioned <1>, wherein a mass ratio of the component (A) to the component (C), (A)/(C), is preferably from 0.03 to 2, and more preferably from 0.04 to 0.25.
<3> The skin cleansing composition according to the aforementioned <1> or <2>, wherein, in the component (A), in the formula (1), the average value of n is preferably from 2.8 to 3.1.
<4> The skin cleansing composition according to any one of the aforementioned <1> to <3>, wherein, the component (A) has, in the formula (1), preferably, a ratio of (the mass of a component in which n = 0) : (the mass of a component in which n = 1) : (the mass of a component in which n = 2) : (the mass of a component in which n = 3) : (the mass of a component in which n = 4) = 1 : 0.99 to 3.50 : 0.89 to 3.00 : 0.76 to 3.00 : 0.63 to 1.52 in a component having the alkyl chain length contained with the highest content in a composition of R¹.
<5> The skin cleansing composition according to any one of the aforementioned <1> to <7>, wherein, in the component (A), in the formula (1), R¹ preferably contains two or more alkyl groups, and the content of a component having an alkyl chain length which is contained in the highest content is preferably 55% by mass or more and less than 97% by mass, more preferably from 60 to 95% by mass, and even more preferably from 70 to 95% by mass.
<6> The skin cleansing composition according to any one of the aforementioned <1> to <5>, wherein, preferably, the component (A) contains, in the formula (1), a component in which n = 0 in an amount of 9.9% by mass or more and less than 12% by mass and has a ratio of (the mass of a component in which n = 0) : (the mass of a component in which n = 1) : (the mass of a component in which n = 2) : (the mass of a component in which n = 3) : (the mass of a component in which n = 4) = 1 : 1.53 to 1.87 : 1.59 to 2.25 : 1.33 to 2.16 : 1.14 to 1.52 in a component having the alkyl chain length contained with the highest content in a composition of R¹, or contains a component in which n = 0 in an amount of from 12 to 17% by mass and has a ratio of (the mass of a component in which n = 0) : (the mass of a component in which n = 1) : (the mass of a component in which n = 2) : (the mass of a component in which n = 3) : (the mass of a component in which n = 4) = 1 : 0.99 to 1.34 : 0.89 to 1.40 : 0.76 to 1.23 : 0.63 to 1 in a component having the alkyl chain length contained with the highest content in a composition of R¹.
<7> The skin cleansing composition according to any one of the aforementioned <1> to <6>, wherein the content of the component (A) is preferably from 1 to 5% by mass, more preferably from 1 to 4% by mass, and even more preferably from 2 to 4% by mass of the total composition.
<8> The skin cleansing composition according to any one of the aforementioned <1> to <7>, wherein the component (B) is preferably hydroxyethylcellulose, hydroxypropylcellulose, hydroxyethyl methyl cellulose, or hydroxypropyl methyl cellulose, and the average molecular weight is preferably 200,000 or more, more preferably 350,000 or more, even more preferably 500,000 or more, further preferably 650,000 or more, and preferably 3,000,000 or less, more preferably 2,000,000 or less, and even more preferably 1,600,000 or less.
<9> The skin cleansing composition according to any one of the aforementioned <1> to <8>, wherein the component (B) is preferably hydroxyethylcellulose or hydroxypropyl methyl cellulose, the average degree of substitution is preferably from 0.5 to 2, and the average molecular weight is preferably from 650,000 to 1,600,000.
<10> The skin cleansing composition according to any one of the aforementioned <1> to <9>, wherein the content of the component (B) is preferably from 0.2 to 0.8% by mass, more preferably from 0.3 to 0.8% by mass, and even more preferably from 0.3 to 0.5% by mass.
<11> The skin cleansing composition according to any one of the aforementioned <1> to <10>, wherein the component (C) is preferably a fatty acid salt or alkyl (ether) sulfate.
<12> The skin cleansing composition according to any one of the aforementioned <1> to <11>, wherein the component (C) is preferably a fatty acid salt represented by the formula (2):

   R²-COOY (2)

   wherein, R² represents a linear or branched alkyl group or alkenyl group having 9 to 21 carbon atoms and Y represents alkali metal, ammonium, alkanolamine-derived ammonium, or a basic amino acid.
<13> The skin cleansing composition according to any one of the aforementioned <1> to <11>, wherein the component (C) is preferably alkyl (ether) sulfate represented by the formula (3):

   R³-O-(CH₂CH₂O)ₚ-SO₃-Z (3)

   wherein, R³ represents a linear or branched alkyl group or alkenyl group having 8 to 22 carbon atoms, p represents a number of from 0 to 20, the average number of moles added is from 0 to 3, and Z represents a hydrogen atom or a cation selected from alkali metal, alkaline earth metal, ammonium, alkyl ammonium, alkanol ammonium, and glucammonium.
<14> The skin cleansing composition according to the aforementioned <13>, wherein, in the component (C), in the formula (3), R³ is preferably a linear or branched alkyl group or alkenyl group having 12 to 18 carbon atoms, more preferably a linear or branched alkyl group or alkenyl group having 12 to 14 carbon atoms, p is 0 or the average number of moles added is preferably larger than 0 and 3 or less, more preferably larger than 0 and less than 2, and even more preferably from 0.5 to 1.5.
<15> The skin cleansing composition according to any one of the aforementioned <1> to <14>, wherein the content of the component (C) is preferably from 8 to 23% by mass, more preferably from 15 to 23% by mass, and even more preferably from 18 to 21% by mass of the total composition.
<16> The skin cleansing composition according to any one of the aforementioned <1> to <15>, wherein the content of the component (D) is preferably from 30 to 95% by mass, more preferably from 65 to 85% by mass, and even more preferably from 65 to 77% by mass of the total composition.
<17> The skin cleansing composition according to any one of the aforementioned <1> to <16>, further comprising (E) an amphoteric surfactant.
<18> The skin cleansing composition according to the aforementioned <17>, wherein the component (E) is preferably alkyldimethylaminoacetic acid betaine having 12 to 18 carbon atoms, alkyldimethylamine oxide having 12 to 18 carbon atoms, 2-alkyl-N-carboxymethyl-N-hydroxyethyl imidazolinium betaine, fatty acid amidopropyl betaine having 12 to 18 carbon atoms, or alkyl hydroxy sulfobetaine having 12 to 18 carbon atoms, more preferably fatty acid amidopropyl betaine having 12 to 18 carbon atoms or alkyl hydroxy sulfobetaine having 12 to 18 carbon atoms, and even more preferably lauric acid amidopropyl betaine or lauryl hydroxy sulfobetaine.
<19> The skin cleansing composition according to the aforementioned <17> or <18>, wherein the content of the component (E) is preferably from 0.5 to 5% by mass, more preferably from 1.5 to 3.5% by mass, and even more preferably from 1.5 to 2% by mass of the total composition.
<20> The skin cleansing composition according to any one of the aforementioned <1> to <19>, further comprising (F) an alkyl alkanolamide represented by the formula (4): 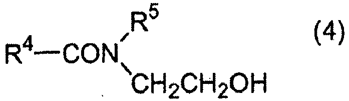 wherein, R⁴CO represents an acyl group having 8 to 20 carbon atoms and R⁵ represents a hydrogen atom, a methyl group, or a hydroxyethyl group.
<21> The skin cleansing composition according to the aforementioned <20>, wherein, in the component (F), in the formula (4), R⁴CO is preferably an acyl group having 10 to 18 carbon atoms and R⁵ is preferably a hydrogen atom or a methyl group.
<22> The skin cleansing composition according to the aforementioned <20> or <21>, wherein the content of the component (F) is preferably from 0.2 to 2% by mass, and more preferably from 0.5 to 1% by mass of the total composition.
<23> A skin cleansing method, comprising applying the skin cleansing composition according to any one of the aforementioned <1> to <22> to a skin area, washing, and then rinsing.

### <Measurement method>

In the present invention, the alkyl composition, the distribution of the moles of EO added, and the ratio of each component of the alkyl ether carboxylic acid were measured by the following analytical method with gas chromatography (GC).

### (GC analytical conditions)

GC instrument; the product of Agilent Technologies, 7890A Column; the product of Agilent Technologies, DB-5 (30 m, an inner diameter of 0.25 mm, a film thickness of 0.25 µm)
Detector; FID
Carrier; helium gas, 1 mL/min
Conditions of temperature rising; temperature is raised at 5°C/min from 100°C to 325°C, and thereafter, maintained at 325°C for 35 minutes.

### (Method of sample pretreatment)

Into 50 mL of methanol, 150 mg of alkyl ether carboxylic acid was dissolved. Also, the cleansing composition was taken in an amount of 150 mg in terms of alkyl ether carboxylic acid equivalent and dissolved in 50 mL of methanol. Also, when the cleansing composition contained a strong anionic surfactant such as polyoxyethylene alkyl ether sulfate, the cleansing composition was collected in such an amount that the strong anionic surfactant was 250 mg or less. From these solutions, 1 mL was taken and applied to a solid phase cartridge (manufactured by Biotage Japan Ltd., Isolute SAX, 1 g, 3 mL, 500-0100-B) which had been conditioned with 4 mL of methanol in advance, and the filtrate was received in a 10 mL round-bottom test tube. Subsequently, the eluate which was eluted with 6 mL of a solution of 4.6 g of formic acid in 100 mL of methanol was also collected in the same test tube. The solution thus collected was set in a block heater heated to 50°C, to which nitrogen gas was blown in, and the solution was concentrated to approximately 1 mL, which was then dried at room temperature by further blowing nitrogen gas. To the resulting product, 2 mL of a diazomethane-ether solution was added, and the resulting solution was left to stand at room temperature for 10 minutes while stirring to carry out derivatization. Subsequently, nitrogen gas was blown in at room temperature and the solution was concentrated to 500 µL or less, to which chloroform was then added to bring the total volume to 500 µL, and the resulting product was subjected to GC analysis.

It is to be noted that the diazomethane-ether solution was prepared by the following procedure using a diazomethane generator (manufactured by Miyamoto Riken Ind. Co., Ltd., GM-50). A first receiver and a second receiver, and the second receiver and a third receiver were connected using a silicone rubber plug and a Teflon (Registered trademark) tube. Into the second receiver, 0.8 g of N-methyl-N'-nitro-N-nitrosoguanidine was collected, to which 2.5 mL of ion exchange water was added. Into the third receiver, 10 mL of tert-butyl methyl ether was collected. The first, second, and third receivers were cooled on ice. Subsequently, the second receiver was fitted with a plastic syringe, into which 3 mL of a solution of 20 g of sodium hydroxide dissolved in 100 mL of ion exchange water was placed. This aqueous solution of sodium hydroxide was slowly added dropwise to generate diazomethane gas, and nitrogen gas was gently blown in from the first receiver side to dissolve the diazomethane gas in tert-butyl methyl ether in the third receiver, whereby a diazomethane-ether solution was obtained.

The following reagents were used in the aforementioned sample pretreatment.
Methanol (manufactured by Kanto Chemical Co., Inc., for high performance liquid chromatography, 25183-1B) Formic acid (manufactured by Wako Pure Chemical Industries, Ltd., special grade chemical, 066-00461) Chloroform (manufactured by Kanto Chemical Co., Inc., CICA first grade, 07278-01) N-Methyl-N'-nitro-N-nitrosoguanidine (manufactured by Kanto Chemical Co., Inc., CICA first grade, 25596-51) Tert-butyl methyl ether (manufactured by Kanto Chemical Co., Inc., CICA special grade, 04418-00)
Sodium hydroxide (manufactured by Wako Pure Chemical Industries, Ltd., special grade, 196-13761)

### <Production Example>

The alkyl ether carboxylic acid of the component (B) used in the framed soap composition of the present invention is obtainable by a production method of alkyl ether carboxylic acid, which comprises reacting ethylene oxide with one or two or more alcohols selected from alcohols having an alkyl group having 4 to 22 carbon atoms to obtain alkyl ethoxylate, and then allowing the alkyl ethoxylate thus obtained to undergo further reactions. Specifically, the alkyl ether carboxylic acid of the component (B) can be produced, for example, as follows. Also, unless otherwise noted, "%" represents % by mass.

### Production Example 1

Into a stainless steel autoclave with stirring and temperature controlling functions, 1144 g (6.14 mol) of lauryl alcohol [trade name: KALCOL 2098, manufactured by Kao Corporation], 60.2 g (0.281 mol) of myristyl alcohol [trade name: KALCOL 4098, manufactured by Kao Corporation], and 2.68 g (0.0478 mol) of potassium hydroxide were placed and dehydration was performed under reduced pressure. Subsequently, 996 g (22.6 mol) of ethylene oxide (EO) was introduced at 155°C and reactions were allowed to proceed at a reaction temperature of 155°C and a reaction pressure of 0.4 MPa for two hours. Upon completion of the reaction, the resulting mixture was stirred for 30 minutes at 80°C under a reduced pressure condition of 6 kPa. Then, after removing unreacted ethylene oxide, nitrogen was introduced to normalize the pressure, and 4.82 g (0.0482 mol) of 90% lactic acid was added into the autoclave, followed by stirring at 80°C for 30 minutes, whereby alkyl ethoxylate having 3.55 moles of EO added (hereinbelow, also referred to as "the produced AE") was obtained.

Into a glass reaction container with stirring and temperature controlling functions and an oxygen gas introduction tube, 90 g (0.2 mol) of the aforementioned product, 16.7 g of a 48% aqueous solution of sodium hydroxide (0.2 mol as sodium hydroxide), 0.9 g of a palladium-platinum-bismuth-based catalyst (activated carbon containing 4% of palladium, 1% of platinum, 5% of bismuth, and 50% of water content), and 494.4 g of water were each placed. While stirring, the liquid temperature was raised to 70°C, and while blowing oxygen in at a ratio of 27 mol% (with respect to the produced AE / hour), catalytic oxidation reactions were carried out at a reaction temperature of 70°C for 3.5 hours. The rate of reaction was 89%.

Upon completion of the reaction, the catalyst was filtered out from the reaction solution to provide an aqueous solution of sodium salt of alkyl ether carboxylic acid. Subsequently, 35% hydrochloric acid was added, and a liquid separation operation was performed to provide alkyl ether carboxylic acid, which will be referred to as EC1.

As a result of gas chromatography analysis, it was found that, in the formula (1), M = H, R¹ had lauryl group / myristyl group at a ratio of 95 / 5, the average carbon number was 12.1, and the average value of n was 2.8, and EC1 contained a component in which n = 0 in an amount of 14.7% by mass, and a component in which n = 1 and a component in which n = 2 in a total amount of 36.1% by mass.

Further, it was also found that the ratio of each of the components having different numbers of moles of EO added, as calculated from the measurement value of the maximum component of the composition of R¹, was as follows; (the mass of a component in which n = 0) : (the mass of a component in which n = 1) : (the mass of a component in which n = 2) : (the mass of a component in which n = 3) : (the mass of a component in which n = 4) = 1 : 1.22 : 1.23 : 1.06 : 0.83.

### Production Example 2

According to Production Example 1, EO was reacted with a raw material containing a mixture of decyl alcohol [trade name: KALCOL 1098, manufactured by Kao Corporation], lauryl alcohol [trade name: KALCOL 2098, manufactured by Kao Corporation], myristyl alcohol [trade name: KALCOL 4098, manufactured by Kao Corporation], and cetyl alcohol [trade name: KALCOL 6098, manufactured by Kao Corporation] at a mass ratio of 10 / 70 / 15 / 5 to provide alkyl ethoxylate having 3.55 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ had decyl group/lauryl group/myristyl group/ palmityl group at a ratio of 10 / 70 / 15 / 5, the average carbon number was 12.3, and the average value of n was 3.3, and the alkyl ether carboxylic acid contained a component in which n = 0 in an amount of 15.2% by mass, and a component in which n = 1 and a component in which n = 2 in a total amount of 31.4% by mass.

Further, it was also found that the ratio of each of the components having different numbers of moles of EO added, as calculated from the measurement value of the maximum component of the composition of R¹, was as follows; (the mass of a component in which n = 0) : (the mass of a component in which n = 1) : (the mass of a component in which n = 2) : (the mass of a component in which n = 3) : (the mass of a component in which n = 4) = 1 : 1.07 : 1.00 : 0.85 : 0.67.

### Production Comparative Example 3

Into a glass reaction container with stirring and temperature controlling functions, 372 g (2.00 mol) of lauryl alcohol was placed, and while stirring, the liquid temperature was raised to 70°C. Then, while adding 256 g (2.20 mol) of sodium monochloroacetate and 88 g (2.20 mol) of sodium hydroxide in divided portions, a reaction was allowed to proceed for five hours. Upon completion of the reaction, the precipitates were filtered out. Subsequently, 35% hydrochloric acid was added for acidification to obtain alkyl ether carboxylic acid (in the formula (1), M = H, R1 was a lauryl group, and n = 0).

### Production Comparative Example 4

According to Production Example 1, EO was reacted with decyl alcohol as a raw material to provide alkyl ethoxylate having 3.55 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ was a decyl group, and the average value of n was 3.1, and the alkyl ether carboxylic acid contained a component in which n = 0 in an amount of 16% by mass, and a component in which n ₌ 1 and a component in which n = 2 in a total amount of 37% by mass.

### Production Example 5

According to Production Example 1, EO was reacted with lauryl alcohol as a raw material to provide alkyl ethoxylate having 3.55 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ was a lauryl group, and the average value of n was 3.1, and the alkyl ether carboxylic acid contained a component in which n = 0 in an amount of 16% by mass, and a component in which n = 1 and a component in which n = 2 in a total amount of 37% by mass.

Further, it was also found that the ratio of each of the components having different numbers of moles of EO added, as calculated from the measurement value of the maximum component of the composition of R¹, was as follows; (the mass of a component in which n = 0) : (the mass of a component in which n = 1) : (the mass of a component in which n = 2) : (the mass of a component in which n = 3) : (the mass of a component in which n = 4) = 1 : 1.19 : 1.13 : 0.94 : 1.

### Production Example 6

According to Production Example 1, EO was reacted with myristyl alcohol as a raw material to provide alkyl ethoxylate having 3.55 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ was a myristyl group, and the average value of n was 3.1, and the alkyl ether carboxylic acid contained a component in which n = 0 in an amount of 16% by mass, and a component in which n = 1 and a component in which n = 2 in a total amount of 37% by mass.

### Production Example 7

According to Production Example 1, EO was added to a raw material containing a mixture of lauryl alcohol and cetyl alcohol at a mass ratio of 20 / 80 to provide alkyl ethoxylate having 3.55 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ had lauryl group / palmityl group at a ratio of 20 / 80, and the average value of n was 3.1, and the alkyl ether carboxylic acid contained a component in which n = 0 in an amount of 16% by mass, and a component in which n = 1 and a component in which n = 2 in a total amount of 37% by mass.

### Production Comparative Example 8

According to Production Example 1, EO was reacted with lauryl alcohol as a raw material to provide alkyl ethoxylate having 4.05 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in the formula (1), M = H, R¹ was a lauryl group, the average value of n was 3.5, and the resulting alkyl ether carboxylic acid contained a component in which n = 0 in an amount of 11.4% by mass, and a component in which n = 1 and a component in which n = 2 in a total amount of 30.6% by mass.

Further, it was also found that the ratio of each of the components having different numbers of moles of EO added, as calculated from the measurement value of the maximum component of the composition of R¹, was as follows; (the mass of a component in which n = 0) : (the mass of a component in which n = 1) : (the mass of a component in which n = 2) : (the mass of a component in which n = 3) : (the mass of a component in which n = 4) = 1 : 1.31 : 1.38 : 1.25 : 1.06.

### Production Example 9

Into a stainless steel autoclave with stirring and temperature controlling functions, 1144 g (6.14 mol) of lauryl alcohol [trade name: KALCOL 2098, manufactured by Kao Corporation], 60.2 g (0.281 mol) of myristyl alcohol [trade name: KALCOL 4098, manufactured by Kao Corporation], and 2.6 g (0.0478 mol) of potassium hydroxide were placed and dehydration was performed under reduced pressure. Subsequently, 718 g (16.3 mol) of ethylene oxide (EO) was introduced at 155°C and a reaction was allowed to proceed at a reaction temperature of 155°C and a reaction pressure of 0.4 MPa for two hours. Upon completion of the reaction, the resulting mixture was cooled and then stirred for 30 minutes at 80°C under a reduced pressure condition of 6 kPa. Then, after removing unreacted ethylene oxide, nitrogen was introduced to normalize the pressure, and 4.82 g (0.0482 mol) of 90% lactic acid was added into the autoclave, followed by stirring at 80°C for 30 minutes, whereby alkyl ethoxylate having 2.55 moles of EO added was obtained.

Into a glass reaction container with stirring and temperature controlling functions, 600 g (2.00 mol) of the aforementioned product was placed, and while stirring, the liquid temperature was raised to 70°C. Then, while adding 256 g (2.20 mol) of sodium monochloroacetate and 88 g (2.20 mol) of sodium hydroxide in divided portions, a reaction was allowed to proceed for five hours. Upon completion of the reaction, 35% hydrochloric acid was added for acidification until pH was 2.8, and the resulting oil layer was collected to obtain alkyl ether carboxylic acid, which will be referred to as EC6.

As a result of gas chromatography analysis, it was found that, in the formula (1), M = H, R¹ had lauryl group / myristyl group at a ratio of 94 / 6, the average carbon number was 12.1, and the average value of n was 3.1, and EC6 contained a component in which n = 0 in an amount of 9.9% by mass, and a component in which n = 1 and a component in which n = 2 in a total amount of 35.4% by mass.

Further, it was also found that the ratio of each of the components having different numbers of moles of EO added, as calculated from the measurement value of the maximum component of the composition of R¹, was as follows; (the mass of a component in which n = 0) : (the mass of a component in which n = 1) : (the mass of a component in which n = 2) : (the mass of a component in which n = 3) : (the mass of a component in which n = 4) = 1 : 1.65 : 1.92 : 1.74 : 1.32.

In the Examples, EC2 was obtained by mixing each of the alkyl ether carboxylic acids produced in Production Examples 5, 6, and 7 at a mass ratio of 78.75 / 15 / 6.25.

In the Examples, EC3 was obtained by mixing each of the alkyl ether carboxylic acids obtained in Production Examples 2 and 3 at a mass ratio of 90 / 10.

In the Examples, EC4 was obtained by mixing each of EC1 obtained in Production Example 1 and the alkyl ether carboxylic acid obtained in Production Example 4 at a mass ratio of 40 / 60.

In the Examples, EC5 was obtained by mixing each of the alkyl ether carboxylic acids obtained in Production Examples 2 and 8 at a mass ratio of 40 / 60.

### [Examples]

The composition of the component (A) used in Examples is as shown in Tables 1 and 2.

Also, for the average numbers of moles of EO added to commercially available alkyl ether carboxylic acids used in Examples (AKYPO RLM45 (manufactured by Kao Corporation)), values provided in the catalogue supplied by each vendor or values posted in the website of each vendor were referred to. Unknown alkyl composition, the amount of a component in which n = 0, and the total amount of a component in which n = 1 and a component in which n = 2 were analyzed by the aforementioned method.

**[Table 1]**

| | R1 (% by mass) | | | | Average carbon number | Average number of moles of EO added | n=0 Content ratio | n=1, 2 Total amount |
|---|---|---|---|---|---|---|---|---|
| | C10 | C12 | C14 | C16 | | | | |
| EC1 | 0 | 95 | 5 | 0 | 12.1 | 2.8 | 14.7% | 36.1% |
| EC2 | 0 | 80 | 15 | 5 | 12.5 | 3.1 | 16.0% | 32.5% |
| EC3 | 8 | 73 | 13.5 | 4.5 | 12.2 | 2.8 | 27.0% | 27.1% |
| EC4 | 60 | 38 | 2 | 0 | 10.8 | 3.2 | 12.5% | 34.8% |
| EC5 | 4 | 88 | 6 | 2 | 12.1 | 3.4 | 13.3% | 31.0% |
| EC6 | 0 | 94 | 6 | 0 | 12.1 | 3.1 | 9.9% | 35.4% |

**[Table 2]**

| | R1 (% by mass) | | | | Average carbon number | Average number of moles of EO added | n=0 Content ratio | n=1, 2 Total amount |
|---|---|---|---|---|---|---|---|---|
| | C10 | C12 | C14 | C16 | | | | |
| 45CA *1 | 0 | 68 | 26 | 6 | 12.8 | 4.5 | 9.6% | 31.2% |

| | | | | | | | | |
|---|---|---|---|---|---|---|---|---|
| *1 Active ingredient of KAOAKYPO RLM-45 (Polyoxyethylene lauryl ether acetic acid) (90%) (manufactured by Kao Corporation) | | | | | | | | |

### Examples 1 to 8 and Comparative Examples 1 to 3

The skin cleansing compositions having the compositions as shown in Table 3 were produced, and the quickness of foaming during washing, foam retentivity when the part to be washed is changed, impression of thickness of foam during washing, soft feeling of the skin during washing, a stop feeling at the end of rinsing, lack of residual feel at the completion of rinsing, and moist feeling after towel drying were evaluated. The results are shown in Table 3 altogether.

### (Production Method)

### (1) Products containing hydroxyethylcellulose:

The components (A) and (C), and when necessary, a 50% aqueous solution of malic acid, and further, ion-exchanged water (in an amount equal to 90% of the total volume added) were mixed and heated to 70°C, and then homogenized. Subsequently, a 48% aqueous solution of potassium hydroxide was added in an amount to provide pH = 9.5, followed by stirring to homogeneity. Further, a dispersion of hydroxyethylcellulose in the remaining ion-exchanged water (25°C) was added. After stirring at 70°C, the component (E), the component (F), and ethylene glycol distearate were added, as needed, followed by stirring to homogeneity. Subsequently, the mixture was cooled to room temperature while stirring, and then, when necessary, fragrance was added, and stirring was continued until homogeneity was achieved, whereby skin cleansing compositions were obtained.

### (2) Products containing hydroxypropyl methyl cellulose:

The entire volume of ion-exchanged water was heated to 70°C, to which hydroxypropyl methyl cellulose was gradually added while stirring to achieve uniform dispersion. The components (A) and (C), and when necessary, a 50% aqueous solution of malic acid were added, and the resulting mixture was stirred until it reached 70°C. Subsequently, a 48% aqueous solution of potassium hydroxide was added in an amount to provide pH = 9.5, followed by stirring to homogeneity. The component (E), the component (F), and ethylene glycol distearate were added, as needed, followed by stirring to homogeneity. Subsequently, the mixture was cooled to room temperature while stirring, and then, when necessary, fragrance was added, and stirring was continued until homogeneity was achieved, whereby skin cleansing compositions were obtained.

### (Evaluation Method)

After moistening the right and left forearms with tap water of 30°C, each skin cleansing composition (1 g) was poured onto one hand and directly applied to the forearm (from the elbow to the wrist) of the other arm. Subsequently, lather was produced by moving the palm back and forth 20 times in the axial direction of the arm. At this time, quickness of foaming during washing was evaluated based on the state of the foam on the forearm. Also, based on the sensation felt by the forearm and by the palm from the 16^{th} to 20^{th} strokes of a series of 20 back-and-forth movements, the impression of thickness of foam and soft feeling of the skin during washing were evaluated.

Subsequently, rinsing was performed with tap water of 30°C. At this time, rinsing was performed while rubbing the forearm and the palm against each other, and lack of residual feel at the completion of rinsing and a feeling of the palm being stopped at the completion of rinsing (a stop feeling at the end of rinsing) were evaluated. Further, a moist feeling of the skin after towel drying was evaluated.

Each evaluation was made based on the following criteria, and the results were shown as the average values of three expert panelists.
(1) Quickness of foaming during washing:
   5; Felt foaming was quick.
   4; Felt foaming was a little quick.
   3; No opinion.
   2; Felt foaming was a little slow.
   1; Felt foaming was slow.
(2) Impression of thickness of foam during washing:
   5; Felt the foam was thick.
   4; Felt the foam was a little thick.
   3; No opinion.
   2; Felt the foam was less thick.
   1; Felt the foam was not thick.
(3) Soft feeling of the skin during washing:
   5; Felt the skin was soft.
   4; Felt the skin was a little soft.
   3; No opinion.
   2; Felt the skin was a little hard with friction.
   1; Felt the skin was hard with friction.
(4) A stop feeling at the end of rinsing:
   5; A stop feeling was strongly felt at the end of rinsing.
   4; A stop feeling was slightly strongly felt at the end of rinsing.
   3; A stop feeling was moderately felt at the end of rinsing.
   2; A stop feeling was slightly weakly felt at the end of rinsing.
   1; A stop feeling was weakly felt at the end of rinsing.
(5) Lack of residual feel at the completion of rinsing:
   5; Felt residual feel was absent at the completion of rinsing.
   4; Felt residual feel was almost absent at the completion of rinsing.
   3; No opinion.
   2; Felt residual feel was slightly present at the completion of rinsing.
   1; Felt residual feel was present at the completion of rinsing.
(6) Moist feeling of the skin after towel drying:
   5; Moist.
   4; A little moist.
   3; No opinion.
   2; A little dry.
   1; Dry.
(7) Foam retentivity when the part to be washed is changed:
   After moistening the right and left forearms with tap water of 30°C, each skin cleansing composition (1 g) was poured onto one hand and directly applied to the forearm of the other arm. Subsequently, lather was produced by moving the palm back and forth 20 times horizontally in the direction of the arm. After completing the 20 back-and-forth movements, foam spread over the forearm was collected in the palm of the same arm, and the foam was then transferred to the forearm of the other arm. Foaming was performed again by moving the palm along the arm horizontally in the direction of the arm. Based on the state of the foam on the forearm after 20 back-and-forth movements of the palm, re-foaming properties were evaluated based on the following criteria. Each evaluation was made based on the following criteria, and the results were shown as the average values of three expert panelists.
      5; Compared to the volume of foam immediately after transfer of the foam, the volume of foam increased in the last half of the test.
      4; Compared to the volume of foam immediately after transfer of the foam, the volume of foam slightly increased in the last half of the test.
      3; There was no change in the volume of foam between immediately after transfer of the foam and the last half of the test.
      2; Compared to the volume of foam immediately after transfer of the foam, the volume of foam slightly decreased in the last half of the test.
      1; Compared to the volume of foam immediately after transfer of the foam, the volume of foam decreased in the last half of the test.

**[Table 3]**

| Component (% by mass) | | Example | | | | | | | | Comparative Example | | |
|---|---|---|---|---|---|---|---|---|---|---|---|---|
| | | 1 | 2 | 3 | 4 | 5 | 6 | 7 | 8 | 1 | 2 | 3 |
| A | EC1 | 4 | 4 | 4 | 4 | | | | | | | |
| | EC6 | | | | | 4 | 4 | 6 | 3 | | | |
| | Polyoxyethylene (4.5) lauryl ether acetic acid *1 | | | | | | | | | 4 | 4 | |
| B | Hydroxyethylcellulose *2 | 0.8 | | 0.8 | | 0.8 | 0.8 | 0.8 | 0.8 | 0.8 | | 0.8 |
| | Hydroxypropyl methyl cellulose *3 | | 0.8 | | 0.8 | | | | | | 0.8 | |
| C | Lauric acid | 4 | 4 | | | 4 | | 3 | 6 | 4 | 4 | 4 |
| | Sodium polyoxyethylene (2) lauryl ether sulfate *4 | | | 4 | 4 | | 4 | | | | | |
| | 48% Potassium hydroxide | q.s. | q.s. | q.s. | q.s. | q.s. | q.s. | q.s. | q.s. | q.s. | q.s. | q.s. |
| | 50% Malic acid | 0.8 | 0.8 | 0.8 | 0.8 | 0.8 | 0.8 | 1.2 | 0.6 | 0.6 | 0.6 | 0.6 |
| D | Ion-exchanged water | Balance | Balance | Balance | Balance | Balance | Balance | Balance | Balance | Balance | Balance | Balance |
| | Total | 100 | 100 | 100 | 100 | 100 | 100 | 100 | 100 | 100 | 100 | 100 |
| | pH | 9.5 | 9.5 | 9.5 | 9.5 | 9.5 | 9.5 | 9.5 | 9.5 | 9.5 | 9.5 | 9.5 |
| | A/C | 1 | 1 | 1 | 1 | 1 | 1 | 2 | 0.5 | - | - | - |
| | Quickness of foaming during washing | 3.7 | 3.7 | 4.7 | 4.7 | 3.7 | 3.7 | 3.7 | 4.7 | 1.0 | 1.0 | 3.7 |
| | Foam retentivity when the part to be washed is changed | 4.7 | 4.3 | 3.7 | 3.7 | 3.7 | 4.7 | 3.7 | 3.0 | 1.7 | 1.7 | 2.0 |
| | Impression of thickness of foam during washing | 4.0 | 4.0 | 3.7 | 3.7 | 4.0 | 3.7 | 4.0 | 4.0 | 1.0 | 1.0 | 3.7 |
| | Soft feeling of the skin during washing | 4.7 | 4.7 | 4.0 | 4.0 | 4.7 | 4.0 | 4.0 | 4.7 | 2.0 | 2.0 | 2.0 |
| | Stop feeling at the end of rinsing | 4.0 | 4.0 | 3.0 | 3.0 | 4.0 | 3.0 | 3.7 | 4.7 | 2.3 | 2.3 | 5.0 |
| | Lack of residual feel at the completion of rinsing | 4.0 | 4.0 | 3.7 | 3.7 | 4.0 | 4.0 | 4.0 | 4.0 | 2.3 | 2.0 | 2.0 |
| | Moist feeling after towel drying | 4.3 | 4.3 | 4.0 | 4.0 | 4.3 | 4.0 | 4.0 | 4.0 | 4.0 | 4.0 | 2.0 |

| | | | | | | | | | | | | |
|---|---|---|---|---|---|---|---|---|---|---|---|---|
| *1 Active ingredient of KAOAKYPO RLM-45 (Polyoxyethylene lauryl ether acetic acid) (90%) (manufactured by Kao Corporation) *2 CELLOSIZE QP52000H (manufactured by The Dow Chemical Company), an average molecular weight of 1,500,000 *3 BENECEL K35M (manufactured by Ashland Inc.), an average molecular weight of approximately 690,000 *4 Active ingredient of EMAL 227 (27%) (manufactured by Kao Corporation) | | | | | | | | | | | | |

### Examples 9 to 19 and Comparative Examples 4 to 6

In a similar manner to Examples 1 to 8, the skin cleansing compositions having the compositions as shown in Table 4 were produced, and the quickness of foaming during washing, impression of thickness of liquid at the application of the composition, impression of thickness of foam during washing, soft feeling of the skin during washing, ease of spreading of foam during washing, and moist feeling after towel drying were evaluated. The results are shown in Table 4 altogether.

### (Evaluation Method)

After moistening the whole body with tap water of 40°C, each skin cleansing composition (5 g) was poured onto one hand and the liquid was spread over the palm by rubbing both palms together three times. Subsequently, lather was produced while the composition was directly applied to the forearm. At this time, it was evaluated the impression of thickness of liquid as felt by the palm at the application of the composition. Subsequently, the foam was spread to the upper arm, chest, abdomen, and legs by rubbing with the palm. At this time, ease of spreading of foam was evaluated.

Each evaluation was made based on the following criteria, and the results were shown as the average values of three expert panelists.
(1) Quickness of foaming during washing:
   5; Felt foaming was quick.
   4; Felt foaming was a little quick.
   3; No opinion.
   2; Felt foaming was a little slow.
   1; Felt foaming was slow.
(2) Impression of thickness of liquid at the application of the composition:
   5; Felt the liquid was very thick.
   4; Felt the liquid was thick.
   3; No opinion.
   2; Felt the liquid was less thick.
   1; Felt the liquid was not thick.
(3) Impression of thickness of foam during washing:
   5; Felt the foam was thick.
   4; Felt the foam was a little thick.
   3; No opinion.
   2; Felt the foam was less thick.
   1; Felt the foam was not thick.
(4) Soft feeling of the skin during washing:
   5; Felt the skin was soft.
   4; Felt the skin was a little soft.
   3; No opinion.
   2; Felt the skin was a little hard with friction.
   1; Felt the skin was hard with friction.
(5) Ease of spreading of foam during washing:
   5; Felt the foam was easily spread.
   4; Felt the foam was slightly easily spread.
   3; No opinion.
   2; Felt the foam was slightly difficult to be spread.
   1; Felt the foam was difficult to be spread.
(6) Moist feeling of the skin after towel drying:
   5; Moist.
   4; A little moist.
   3; No opinion.
   2; A little dry.
   1; Dry.

**[Table 4]**

| Component (% by mass) | | Example | | | | | | | | | | | Comparative Example | | |
|---|---|---|---|---|---|---|---|---|---|---|---|---|---|---|---|
| | | 9 | 10 | 11 | 12 | 13 | 14 | 15 | 16 | 17 | 18 | 19 | 4 | 5 | 6 |
| A | EC6 | 2 | 2 | 2 | 1 | 2 | 4 | 2 | 3.3 | 2 | 0.7 | 1 | | 8 | 2 |
| B | Hydroxyethylcellulose *2 | 0.3 | 0.3 | 0.3 | 0.3 | 0.5 | 0.3 | 0.3 | | 0.3 | 0.3 | 0.3 | 0.3 | 0.3 | 1.5 |
| | Hydroxypropyl methyl cellulose *3 | | | | | | | | 0.3 | | | | | | |
| C | Lauric acid | 7 | 6.5 | 7 | 16 | 16 | 6 | 7 | 10.5 | 12 | 16 | 16 | 7 | 3.5 | 7 |
| | Myristic acid | 8 | 8 | 8 | 5 | 5 | 7 | 8 | 3.5 | 6.5 | 5 | 5 | 8 | 4 | 8 |
| | Palmitic acid | 1 | 1.5 | 1 | 1 | 1 | 1 | 1 | 1 | I | 1 | 1 | 1 | 0.5 | 1 |
| | Sodium polyoxyethylene (2) lauryl ether sulfate *4 | 2 | 2 | 2 | 0.5 | 0.5 | 2 | 2 | 1.1 | 0.8 | 0.5 | 0.5 | 2 | 2 | 2 |
| E | Lauryl hydroxy sulfobetaine *6 | 1.8 | 1.8 | 1.8 | 1.8 | 1.8 | 1.8 | | 3.3 | 3 | 1.8 | 1.6 | 1.8 | 1.8 | 1.8 |
| F | Lauric acid monoethanolamide *7 | | | 1 | | | | | | | | | | | |
| | 48% Potassium hydroxide | q.s. | q.s. | q.s. | q.s. | q.s. | q.s. | q.s. | q.s. | q.s. | q.s. | q.s. | q.s. | q.s. | q.s. |
| | Fragrance | 1 | 1 | 1 | 1 | 1 | 1 | 1 | 1 | 1 | 1 | 1 | 1 | 1 | 1 |
| D | Ion-exchanged water | Balance | Balance | Balance | Balance | Balance | Balance | Balance | Balance | Balance | Balance | Balance | Balance | Balance | Balance |
| | Total | 100 | 100 | 100 | 100 | 100 | 100 | 100 | 100 | 100 | 100 | 100 | 100 | 100 | 100 |
| | pH | 9.5 | 9.5 | 9.5 | 9.5 | 9.5 | 9.5 | 9.5 | 9.5 | 9.5 | 9.5 | 9.5 | 9.5 | 9.5 | 9.5 |
| | A/C | 0.11 | 0.11 | 0.11 | 0.044 | 0.089 | 0.25 | 0.11 | 0.20 | 0.10 | 0.031 | 0.044 | - | 0.8 | 0.11 |
| | Quickness of foaming during washing | 4.3 | 4.0 | 5.0 | 5.0 | 5.0 | 4.0 | 4.0 | 4.7 | 4.7 | 5.0 | 4.7 | 2.7 | 2.0 | 2.3 |
| | Impression of thickness of liquid at the application of the agent | 4.0 | 4.3 | 5.0 | 4.0 | 4.0 | 4.0 | 4.0 | 4.3 | 4.3 | 4.0 | 4.0 | 3.0 | 2.7 | 4.0 |
| | Impression of thickness of foam during washing | 4.7 | 5.0 | 5.0 | 5.0 | 5.0 | 4.7 | 4.3 | 4.7 | 4.3 | 4.3 | 4.3 | 3.0 | 3.0 | 3.7 |
| | Soft feeling of the skin during washing | 4.0 | 4.0 | 4.3 | 4.3 | 4.0 | 4.7 | 4.0 | 4.7 | 4.7 | 4.0 | 4.0 | 2.3 | 3.0 | 4.0 |
| | Ease of spreading of foam during washing | 4.3 | 4.3 | 4.7 | 4.7 | 4.7 | 4.7 | 4.0 | 4.3 | 4.7 | 4.3 | 4.3 | 2.3 | 2.3 | 2.3 |
| | Moist feeling after towel drying | 4.0 | 4.3 | 4.7 | 4.0 | 4.0 | 4.7 | 4.0 | 4.7 | 4.0 | 4.0 | 4.0 | 2.7 | 4.7 | 4.0 |

| | | | | | | | | | | | | | | | |
|---|---|---|---|---|---|---|---|---|---|---|---|---|---|---|---|
| *2 CELLOSIZE QP52000H (manufactured by The Dow Chemical Company), an average molecular weight of 1,500,000 *3 BENECEL K35M (manufactured by Ashland Inc.), an average molecular weight of approximately 690,000 *4 Active ingredient of EMAL 227 (polyoxyethylene lauryl ether sulfuric acid) (27%) (manufactured by Kao Corporation) *6 Active ingredient of AMPHITOL 20HD (lauryl hydroxy sulfobetaine) (30%) (manufactured by Kao Corporation) *7 AMINON C01S (lauric acid monoethanolamide) (manufactured by Kao Corporation) | | | | | | | | | | | | | | | |

### Examples 20 to 35

In a similar manner to Examples 1 to 8, the cleansing compositions having the compositions as shown in Table 5 were produced.

All of the cleansing compositions produced maintained rich lather even in the last half of washing of the whole body with hands, and the liquids were easily spread, and once the compositions began to foam, the foam was easily spread with excellent smoothness. Also, the compositions gave the impression of thickness of foam, and thus it was felt that the skin was soft during washing. Further, a sleek, smooth feel on the skin was achieved during rinsing, and after towel drying, a soft and smooth feel on the skin was obtained.

**[Table 5]**

| Component (% by mass) | | Example | | | | | | | | | | | | | | | |
|---|---|---|---|---|---|---|---|---|---|---|---|---|---|---|---|---|---|
| | | 20 | 21 | 22 | 23 | 24 | 25 | 26 | 27 | 28 | 29 | 30 | 31 | 32 | 33 | 34 | 35 |
| A | EC1 | 2 | | | | | | | | | | | | | | | |
| | EC2 | | 2 | | | | | | | | | | | | | | |
| | EC3 | | | 2 | | | | | | | | | | | | | |
| | EC4 | | | | 2 | | | | | | | | | | | | |
| | EC5 | | | | | 2 | | | | | | | | | | | |
| | EC6 | | | | | | 2 | 2 | 2 | 1 | 1 | 1 | 1.5 | 3.3 | 3.3 | 3.3 | 3.3 |
| B | Hydroxyethylcellulose *2 | 0.3 | 0.3 | 0.3 | 0.3 | 0.3 | 0.3 | 0.1 | 0.5 | 0.3 | 0.3 | | 0.3 | | | | |
| | Hydroxyethylcellulose *8 | | | | | | | | | | | 0.3 | | | | | |
| | Hydroxypropyl methyl cellulose *3 | | | | | | | | | | | | | 0.3 | | | 0.2 |
| | Hydroxypropyl methyl cellulose *9 | | | | | | | | | | | | | | 0.3 | | |
| | Hydroxypropyl methyl cellulose *10 | | | | | | | | | | | | | | | 0.3 | |
| C | Lauric acid | 7 | 7 | 7 | 7 | 7 | 7 | 7 | 7 | 16 | 16 | 16 | 11 | 10.5 | 10.5 | 10.5 | 10.5 |
| | Myristic acid | 8 | 8 | 8 | 8 | 8 | 8 | 8 | 8 | 5 | 5 | 5 | 6 | 3.5 | 3.5 | 3.5 | 3.5 |
| | Palmitic acid | 1 | 1 | 1 | 1 | 1 | 1 | 1 | 1 | 1 | 1 | 1 | | 1 | | | 1 |
| | Sodium polyoxyethylene (2) lauryl ether sulfate *4 | 2 | 2 | 2 | 2 | 2 | 2 | 2 | 2 | 0.5 | 0.5 | 0.5 | 1.1 | 0.8 | 0.8 | 0.8 | 0.8 |
| E | Lauric acid amidopropyl betaine *5 | | | | | | | | | | 1.8 | 1.8 | | | | | |
| | Lauryl hydroxy sulfobetaine *6 | 1.8 | 1.8 | 1.8 | 1.8 | 1.8 | 1.8 | 1.8 | 1.8 | 0.5 | | | 1.8 | 3.3 | 3.3 | 3.3 | 3.3 |
| F | Lauric acid monoethanolamide *7 | 1 | 1 | 1 | 1 | 1 | 1 | 1 | 1 | | | | 0.5 | 0.6 | 0.6 | 0.6 | 0.6 |
| | Ethylene glycol distearate | 2 | 2 | 2 | 2 | 2 | 2 | 2 | 2 | 2 | 2 | 2 | 2 | 1.5 | 1.5 | 1.5 | 1.5 |
| | 48% Potassium hydroxide | q.s. | q.s. | q.s. | q.s. | q.s. | q.s. | q.s. | q.s. | q.s. | q.s. | q.s. | q.s. | q.s. | q.s. | q.s. | q.s. |
| | Fragrance | 1 | 1 | 1 | 1 | 1 | 1 | | 1 | 1 | 1 | 1 | 1 | 1 | 1 | 1 | 1 |
| D | Ion-exchanged water | Balance | Balance | Balance | Balance | Balance | Balance | Balance | Balance | Balance | Balance | Balance | Balance | Balance | Balance | Balance | Balance |
| | Total | 100 | 100 | 100 | 100 | 100 | 100 | 100 | 100 | 100 | 100 | 100 | 100 | 100 | 100 | 100 | 100 |
| | pH | 9.5 | 9.5 | 9.5 | 9.5 | 9.5 | 9.5 | 9.5 | 9.5 | 9.5 | 9.5 | 9.5 | 9.5 | 9.5 | 9.5 | 9.5 | 9.5 |
| | A/C | 0.11 | 0.11 | 0.11 | 0.11 | 0.11 | 0.11 | 0.11 | 0.11 | 0.044 | 0.044 | 0.044 | 0.083 | 0.21 | 0.21 | 0.21 | 0.21 |

| | | | | | | | | | | | | | | | | | |
|---|---|---|---|---|---|---|---|---|---|---|---|---|---|---|---|---|---|
| *2 CELLOSIZE QP52000H (manufactured by The Dow Chemical Company), an average molecular weight of 1,500,000 *3 BENECEL K35M (manufactured by Ashland Inc.), an average molecular weight of approximately 690,000 *4 Active ingredient of EMAL 227 (polyoxyethylene lauryl ether sulfuric acid) (27%) (manufactured by Kao Corporation) *5 Active ingredient of AMPHITOL 20AB (lauric acid amidopropyl betaine) (30%) (manufactured by Kao Corporation) *6 Active ingredient of AMPHITOL 20HD (lauryl hydroxy sulfobetaine) (30%) (manufactured by Kao Corporation) *7 AMINON C01S (lauric acid monoethanolamide) (manufactured by Kao Corporation) *8 DAICEL HEC SE900 (manufactured by Daicel FineChem Ltd.), an average molecular weight of 1,560,000 *9 METOLOSE 60SH-4000 (manufactured by Shin-Etsu Chemical Co., Ltd.), an average molecular weight of 350,000 *10 METOLOSE 60SH-10000 (manufactured by Shin-Etsu Chemical Co., Ltd.), an average molecular weight of 380,000 | | | | | | | | | | | | | | | | | |

## Claims

1. A skin cleansing composition, comprising the following components (A), (B), (C) and (D):
(A) from 0.5% to 6% by mass of an alkyl ether carboxylic acid or a salt thereof represented by the formula (1):
R¹-O-(CH₂CH₂O)ₙ-CH₂-COOM (1)
wherein, R¹ represents an alkyl group having 4 to 22 carbon atoms, n represents a number of from 0 to 20, wherein the average value of n is 2.8 to 3.4, and M represents a hydrogen atom, alkali metal, alkaline earth metal, ammonium, or organic ammonium,
wherein, R¹ has an average carbon number of from 10.8 to 12.8,
and wherein, the alkyl ether carboxylic acid or the salt thereof contains a component in which n = 0 in an amount of more than 9.6% by mass and 27% by mass or less, and a component in which n = 1 and a component in which n = 2 in a total amount of 21% by mass or more and less than 40% by mass,
(B) from 0.1 to 1% by mass of a cellulose having a hydroxyethyl group or a hydroxypropyl group added,
(C) from 3 to 30% by mass of an anionic surfactant other than the component (A), and
(D) water.

2. The skin cleansing composition according to claim 1,
wherein a mass ratio of the component (A) to the component (C), (A)/(C), is from 0.03 to 2.

3. The skin cleansing composition according to claim 1 or 2,
wherein, in the component (A), in the formula (1), R¹ represents an alkyl group having 8 to 16 carbon atoms and has the average carbon number of 10.8 to 12.5, the content of a component in which n = 0 is from 9.9 to 16% by mass, and the total content of a component in which n = 1 and a component in which n = 2 is from 27 to 36.5% by mass.

4. The skin cleansing composition according to any one of claims 1 to 3, wherein, in the component (A), in the formula (1), (the mass of a component in which n = 0) : (the mass of a component in which n = 1) : (the mass of a component in which n = 2) : (the mass of a component in which n = 3) : (the mass of a component in which n = 4) = 1 : 0.99 to 3.50 : 0.89 to 3.00 : 0.76 to 3.00 : 0.63 to 1.52 in a component having the alkyl chain length contained with the highest content in a composition of R¹.

5. The skin cleansing composition according to any one of claims 1 to 4, wherein, in the component (A), in the formula (1), R¹ contains two or more alkyl groups, and the content of a component having an alkyl chain length which is contained in the highest content is 55% by mass or more and less than 97% by mass.

6. The skin cleansing composition according to any one of claims 1 to 5, which the component (A) contains, in the formula (1), a component in which n = 0 in an amount of 9.9% by mass or more and less than 12% by mass and has a ratio of (the mass of a component in which n = 0) : (the mass of a component in which n = 1) : (the mass of a component in which n = 2) : (the mass of a component in which n = 3) : (the mass of a component in which n = 4) of 1 _{:} 1.53 to 1.87 : 1.59 to 2.25 : 1.33 to 2.16 : 1.14 to 1. 52 in a component having the alkyl chain length contained with the highest content in a composition of R¹, or contains a component in which n = 0 in an amount of from 12 to 17% by mass and has a ratio of (the mass of a component in which n = 0) : (the mass of a component in which n = 1) : (the mass of a component in which n = 2) : (the mass of a component in which n = 3) : (the mass of a component in which n = 4) of 1 : 0.99 to 1.34 : 0.89 to 1.40 : 0.76 to 1.23 : 0.63 to 1 in a component having the alkyl chain length contained with the highest content in a composition of R¹.

7. The skin cleansing composition according to any one of claims 1 to 6, further comprising from 0.5 to 5% by mass of (E) an amphoteric surfactant.

8. The skin cleansing composition according to any one of claims 1 to 7, further comprising from 0.2 to 2% by mass of (F) an alkyl alkanolamide represented by the formula (4): 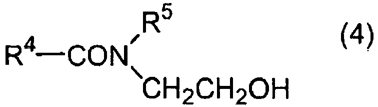 wherein, R⁴CO represents an acyl group having 8 to 20 carbon atoms and R⁵ represents a hydrogen atom, a methyl group, or a hydroxyethyl group.

9. A skin cleansing method, comprising applying the skin cleansing composition according to any one of claims 1 to 8 to a skin area, washing, and then rinsing.

10. Use of the composition according to any one of claims 1 to 8 as a skin cleanser.

## Patentansprüche

1. Hautreinigungszusammensetzung, enthaltend die folgenden Komponenten (A), (B), (C) und (D):
(A) von 0,5 bis 6 Massen-% einer Alkylethercarbonsäure oder eines Salzes davon, dargestellt durch die Formel (1):
R¹-O-(CH₂CH₂O)ₙ-CH₂-COOM (1)
worin R¹ eine Alkylgruppe mit 4 bis 22 Kohlenstoffatomen ist, n eine Zahl von 0 bis 20 ist, worin der Durchschnittswert von n 2,8 bis 3,4 ist, und M ein Wasserstoffatom, Alkalimetall, Erdalkalimetall, Ammonium oder organisches Ammonium ist,
worin R¹ eine durchschnittliche Kohlenstoffzahl von 10,8 bis 12,8 hat und
worin die Alkylethercarbonsäure oder das Salz davon eine Komponente, worin n = 0, in einer Menge von mehr als 9,6 Massen-% und 27 Massen-% oder weniger und eine Komponente, worin n = 1, und eine Komponente, worin n = 2, in einer Gesamtmenge von 21 Massen-% oder mehr und weniger als 40 Massen-% enthält,
(B) von 0,1 bis 1 Massen-% einer Cellulose mit einer zugegebenen Hydroxyethylgruppe oder Hydroxypropylgruppe,
(C) von 3 bis 30 Massen-% eines anderen anionischen Tensides als die Komponente (A) und
(D) Wasser.

2. Hautreinigungszusammensetzung nach Anspruch 1, worin ein Massenverhältnis der Komponente (A) zu der Komponente (C), (A)/(C), von 0,03 bis 2 ist.

3. Hautreinigungszusammensetzung nach Anspruch 1 oder 2, worin in der Komponente (A) in der Formel (1) R¹ eine Alkylgruppe mit 8 bis 16 Kohlenstoffatomen ist und einen durchschnittliche Kohlenstoffzahl von 10,8 bis 12,5 hat, wobei der Gehalt einer Komponente, worin n = 0, von 9,9 bis 16 Massen-% und der Gesamtgehalt einer Komponente, worin n = 1, und einer Komponente, worin n = 2, von 27 bis 36,5 Massen-% ist.

4. Hautreinigungszusammensetzung nach einem der Ansprüche 1 bis 3, worin in der Komponente (A) in der Formel (1) (die Masse einer Komponente, worin n = 0) : (die Masse einer Komponente, worin n = 1) : (die Masse einer Komponente, worin n = 2) : (die Masse einer Komponente, worin n = 3) : (die Masse einer Komponente, worin n = 4) = 1 : 0,99 bis 3,50 : 0,89 bis 3,00 : 0,76 bis 3,00 : 0,63 bis 1,52 in einer Komponente mit der Alkylkettenlänge ist, die mit dem höchsten Gehalt in einer Zusammensetzung von R¹ enthalten ist.

5. Hautreinigungszusammensetzung nach einem der Ansprüche 1 bis 4, worin in der Komponente (A) in der Formel (1) R¹ zwei oder mehr Alkylgruppen enthält und der Gehalt einer Komponente mit einer Alkylkettenlänge, die in dem höchsten Gehalt enthalten ist, 55 Massen-% oder mehr und weniger als 97 Massen-% ist.

6. Hautreinigungszusammensetzung nach einem der Ansprüche 1 bis 5, worin die Komponente (A) in der Formel (1) eine Komponente, worin n = 0, in einer Menge von 9,9 Massen-% oder mehr und weniger als 12 Massen-% enthält und ein Verhältnis von (der Masse einer Komponente, worin n = 0) : (der Masse einer Komponente, worin n = 1) : (der Masse einer Komponente, worin n = 2) : (der Masse einer Komponente, worin n = 3) : (der Masse einer Komponente, worin n = 4) von 1 : 1,53 bis 1,87 : 1,59 bis 2,25 : 1,33 bis 2,16 : 1,14 bis 1,52 in einer Komponente mit der Alkylkettenlänge ist, die mit dem höchsten Gehalt in einer Zusammensetzung von R¹ enthalten ist, oder eine Komponente, worin n = 0, in einer Menge von 12 bis 17 Massen-% enthält und ein Verhältnis von (der Masse einer Komponente, worin n = 0) : (der Masse einer Komponente, worin n = 1) : (der Masse einer Komponente, worin n = 2) : (der Masse einer Komponente, worin n = 3) : (der Masse einer Komponente, worin n = 4) von 1 : 0,99 bis 1,34 : 0,89 bis 1,40 : 0,76 bis 1,23 : 0,63 bis 1 in einer Komponente mit der Alkylkettenlänge hat, die mit dem höchsten Gehalt in einer Zusammensetzung von R¹ enthalten ist.

7. Hautreinigungszusammensetzung nach einem der Ansprüche 1 bis 6, weiterhin enthaltend von 0,5 bis 5 Massen-% von (E) einem amphoteren Tensid.

8. Hautreinigungszusammensetzung nach einem der Ansprüche 1 bis 7, weiterhin enthaltend von 0,2 bis 2 Massen-% von (F) einem Akylalkanolamid mit der Formel (4): 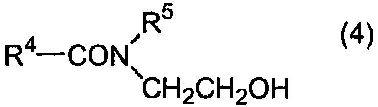 worin R⁴CO eine Acylgruppe mit 8 bis 20 Kohlenstoffatomen und R⁵ ein Wasserstoffatom, eine Methylgruppe oder Hydroxyethylgruppe ist.

9. Hautreinigungsverfahren, enthaltend das Auftragen der Hautreinigungszusammensetzung nach einem der Ansprüche 1 bis 8 auf eine Hautfläche, Waschen und anschließendes Abspülen.

10. Verwendung der Zusammensetzung nach einem der Ansprüche 1 bis 8 als Hautreiniger.

## Revendications

1. Composition de nettoyage de la peau comprenant les composants (A), (B), (C) et (D) suivants :
(A) de 0,5 % à 6 % en masse d'un acide alkyléther carboxylique ou d'un sel de celui-ci représenté par la formule (1) :
R⁻¹-O-(CH₂CH₂O)ₙ-CH₂-COOM (1)
dans laquelle R¹ représente un groupe alkyle ayant 4 à 22 atomes de carbone, n représente un nombre de 0 à 20, dans laquelle la valeur moyenne de n va de 2,8 à 3,4, et M représente un atome d'hydrogène, un métal alcalin, un métal alcalinoterreux, de l'ammonium ou de l'ammonium organique,
dans laquelle R¹ a un nombre de carbones moyen de 10,8 à 12,8,
et dans laquelle l'acide alkyléther carboxylique ou le sel de celui-ci contient un composant dans lequel n = 0 en une quantité de plus de 9,6 % en masse et de 27 % en masse ou moins, et un composant dans lequel n = 1 et un composant dans lequel n = 2 en une quantité totale de 21 % en masse ou plus et de moins de 40 % en masse,
(B) de 0,1 à 1 % en masse d'une cellulose ayant un groupe hydroxyéthyle ou un groupe hydroxypropyle ajouté,
(C) de 3 à 30 % en masse d'un tensioactif anionique autre que le composant (A), et
(D) de l'eau.

2. Composition de nettoyage de la peau selon la revendication 1, dans laquelle un rapport massique du composant (A) au composant (C), (A)/(C), va de 0,03 à 2.

3. Composition de nettoyage de la peau selon la revendication 1 ou 2, dans laquelle, dans le composant (A), dans la formule (1), R¹ représente un groupe alkyle ayant 8 à 16 atomes de carbone et a le nombre de carbones moyen de 10,8 à 12,5, la teneur en un composant dans lequel n = 0 va de 9,9 à 16 % en masse, et la teneur totale en un composant dans lequel n = 1 et un composant dans lequel n = 2 va de 27 à 36,5 % en masse.

4. Composition de nettoyage de la peau selon l'une quelconque des revendications 1 à 3, dans laquelle, dans le composant (A), dans la formule (1), (la masse d'un composant dans lequel n = 0) : (la masse d'un composant dans lequel n = 1) : (la masse d'un composant dans lequel n = 2) : (la masse d'un composant dans lequel n = 3) : (la masse d'un composant dans lequel n = 4) = 1 : 0,99 à 3,50 : 0,89 à 3,00: 0,76 à 3,00 : 0,63 à 1,52 dans un composant ayant la longueur de chaîne alkyle contenue avec la teneur la plus élevée dans une composition de R¹.

5. Composition de nettoyage de la peau selon l'une quelconque des revendications 1 à 4, dans laquelle, dans le composant (A), dans la formule (1), R¹ contient deux groupes alkyle ou plus, et la teneur en un composant ayant une longueur de chaîne alkyle qui est contenue dans la teneur la plus élevée est de 55 % en masse ou plus et de moins de 97 % en masse.

6. Composition de nettoyage de la peau selon l'une quelconque des revendications 1 à 5, dans laquelle le composant (A) contient, dans la formule (1), un composant dans lequel n = 0 en une quantité de 9,9 % en masse ou plus et de moins de 12 % en masse et a un ratio de (la masse d'un composant dans lequel n = 0) : (la masse d'un composant dans lequel n = 1) : (la masse d'un composant dans lequel n = 2) : (la masse d'un composant dans lequel n = 3) : (la masse d'un composant dans lequel n = 4) de 1 : 1,53 à 1,87 : 1,59 à 2,25 : 1,33 à 2,16 : 1,14 à 1,52 dans un composant ayant la longueur de chaîne alkyle contenue avec la teneur la plus élevée dans une composition de R¹, ou contient un composant dans lequel n = 0 en une quantité de 12 à 17 % en masse et a un ratio de (la masse d'un composant dans lequel n = 0) : (la masse d'un composant dans lequel n = 1) : (la masse d'un composant dans lequel n = 2) : (la masse d'un composant dans lequel n = 3) : (la masse d'un composant dans lequel n = 4) de 1 : 0,99 à 1,34 : 0,89 à 1,40: 0,76 à 1,23: 0,63 à 1 dans un composant ayant la longueur de chaîne alkyle contenue avec la teneur la plus élevée dans une composition de R¹.

7. Composition de nettoyage de la peau selon l'une quelconque des revendications 1 à 6, comprenant en outre de 0,5 à 5 % en masse de (E) un tensioactif amphotère.

8. Composition de nettoyage de la peau selon l'une quelconque des revendications 1 à 7, comprenant en outre de 0,2 à 2 % en masse de (F) un alkylalcanolamide représenté par la formule (4) : 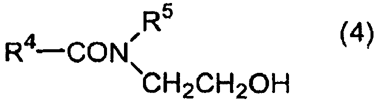 dans laquelle R⁴CO représente un groupe acyle ayant 8 à 20 atomes de carbone et R⁵ représente un atome d'hydrogène, un groupe méthyle ou un groupe hydroxyéthyle.

9. Méthode de nettoyage de la peau, comprenant une application de la composition de nettoyage de la peau selon l'une quelconque des revendications 1 à 8 sur une région de la peau, un lavage puis un rinçage.

10. Utilisation de la composition selon l'une quelconque des revendications 1 à 8 comme nettoyant pour la peau.
